# EUROPEAN PATENT APPLICATION

(11) **EP 1 290 996 A2**
(43) Date of publication of application: **12.03.2003**
(21) Application number: 02405760.6
(22) Date of filing: 03.09.2002
(51) Int. Cl.: A61J 1/00, B65D 75/32, A61M 35/00

(54) **Single-dose pack of substances which cure by absorption or contact**

(30) Priority: 07.09.2001 CH 20011659
(71) Applicant: Fabbri, Doriano, 6596 Gordola (CH); Minacore, Pietra, 6648 Minusio (CH)
(72) Inventor: Fabbri Doriano, 6596 Gordola (CH); Minacore Pietra, 6648 Minusio (CH)
(74) Representative: Fiammenghi-Domenighetti, Delfina

(57) **Abstract**

A single-dose pack (1) of substances (2) capable of performing curative functions through absorption or contact with the injured parts, characterized in that it comprises a sealed flexible envelope (3) of plastics material defining within it a cavity (3c) of elongated shape housing a single dose of the said substances (2) in the liquid state which when converted into the solid state by freezing adopts the shape of an elongated bar of length (L) which is capable of being manipulated in order to reach the said injured part with one end, is described.

## Description

This invention relates to the medical/pharmaceutical sector, in particular it refers to the sector of the production and use of curative substances which perform their functions through absorption or by contact with the injured and/or inflamed parts.
More specifically the invention relates to an innovative single-dose pack for curative substances of the abovementioned type in the liquid state.

The invention has been conceived by operators in the hospital sector who have found that there is a lack of readily-usable means able to relieve pain and at the same time exert a disinfectant and vasoconstrictive effect, especially in infective and inflammatory conditions of the oro-pharyngeal cavity and during corresponding post-operative periods.

In the circumstances mentioned above in particular, experience has shown that the application of ice to wounds or injured parts can alleviate pain. However, ice is currently applied as a gargle, which not all patients are able to practice, or by invasive means or by means which in any event create appreciable discomfort for patients without however exerting any specific disinfectant action.

The inventors of this invention have therefore conceived a single-dose pack which contains a curative substance in the liquid state housed in a cavity thereof of elongated shape. By freezing the said curative substance it takes on the shape of a bar of ice in the solid state and by tearing the enclosure of the pack in question it is possible to manipulate it easily so as to apply one end to the injured parts, so that an antiseptic action accompanied by the analgesic and antihaemorrhagic effect provided by the ice is exerted thereon.

By obtaining the said liquid substances from infusions of natural herbs it is also possible to achieve a pleasant taste and a total lack of undesired effects due to mixing with other medications of a chemical nature which have to be applied to the said injured parts.

Of course in the situation where the substances in the liquid state are natural infusions it is also possible to empty out the contents of the pack, using it as a base for pleasant curative drinks or as a liquid for rinsing and gargling.

The invention therefore provides a functional and extremely simple solution to the problems arising in the medical and/or surgical treatment of the oro-pharyngeal cavity, teeth, etc.

The subject matter of the invention in fact comprises a single-dose container for substances capable of exerting curative functions as described in appended claim 1.

A more detailed description of a preferred embodiment of the single-dose pack according to the invention and the nature of the substance in the liquid state contained therein will now be provided.

In providing this description reference will also be made to the appended drawings which show:
- in Figure 1 a perspective view of the said embodiment of the single-dose pack according to the invention,
- in Figure 2 a transverse cross-section thereof,
- in Figure 3 a perspective view of the single-dose pack with the wrapping torn so that the curative substances are frozen in the form of a bar projecting therefrom and can be manipulated to place one end thereof on the injured parts.

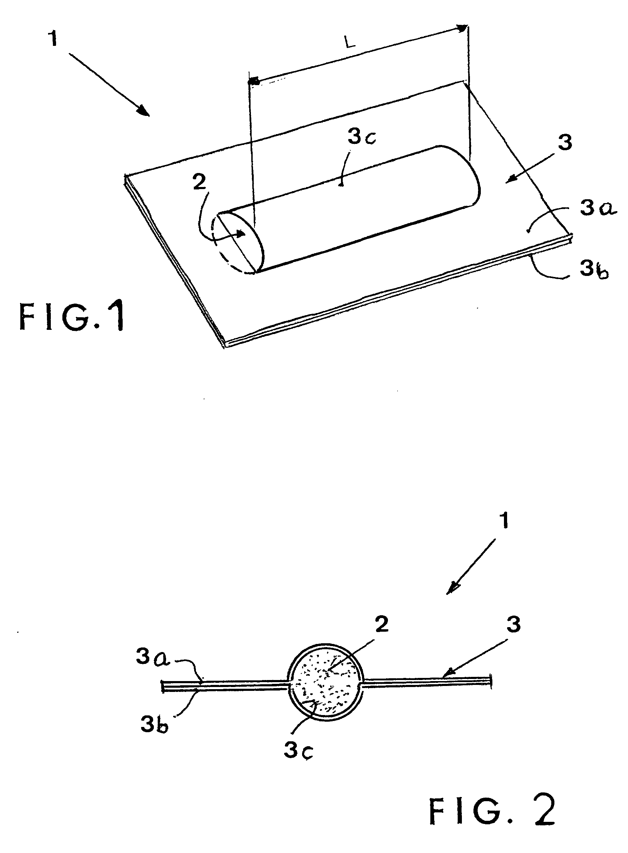

Figures 1 and 2 are first considered. These show how a single-dose pack 1 according to the invention essentially comprises a flat envelope 3 formed from two superimposed sheets 3a, 3b of flexible plastics material.

The two sheets 3a, 3b are attached together by known means, for example by heat welding, after being deformed, again in ways known to those skilled in the art, in such a way that when in place together they form a cavity 3c of elongated shape which is designed to receive within it curative substances 2 when they are in the liquid state. In the embodiment shown said cavity 3c has an elongated cylindrical shape, but its transverse cross-section may be not circular but elliptical, square, etc.

It is important that this cavity 3c has a preset length L, for example 12 - 15 cm, the purpose of which will be explained below.

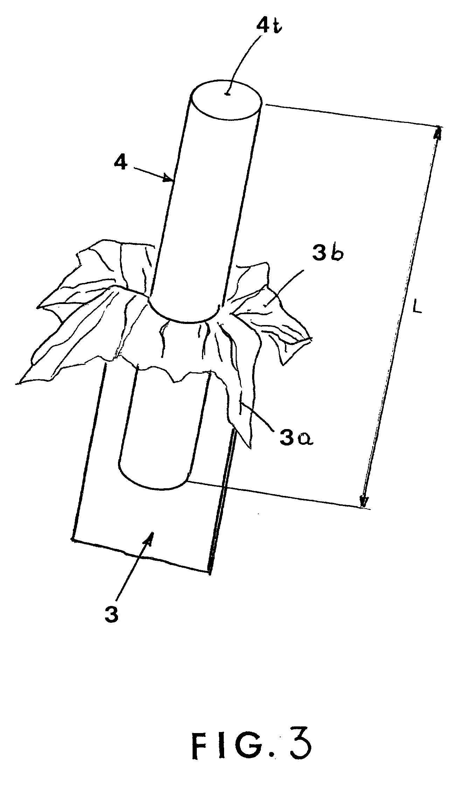

If the said substances 2 are changed from the liquid state to the solid state by freezing, they form a bar 4 (see in this respect Figure 3) in the shape of this cylindrical casing and if envelope 3 is cut or torn as shown in Figure 3 it can be gripped by what is left of envelope 3, readily manipulating it so that its free end 4t can be applied to the injured or inflamed parts, thus exerting the desired soothing and/or curative actions upon it.

As already mentioned, there is nothing to prevent substances 2 contained in envelope 3 from being used without being previously frozen as a basis for a herbal tea (when its composition so permits) or as a liquid for rinsing and gargling.

Again to emphasise this versatility in the use of a single-dose container 1 according to the invention, the inventors suggest that the said curative substances 2 in the liquid state should be obtained from natural infusions of herbs and flowers, with the addition of sweetening and/or colouring agents according to use.

It is not necessary to add any preservative if the said liquid substances 2 are pasteurized before being placed in envelope 3, or if they are subjected to other treatments having a similar effect.

In a first embodiment the aforesaid substances 2 in the liquid state comprise a mixture of the following infusions:
- an infusion of plantain, approximately 25% by weight,
- an infusion of mallow, approximately 25% by weight,
- an infusion of camomile, approximately 25% by weight,
- an infusion of sage, approximately 12.5% by weight,
- an infusion of thyme, approximately 6.25% by weight,
- with added lemon juice, approximately 6.25% by weight.

In another embodiment with a view to achieving a better flavour for substances 2, the latter comprise a mixture of the following infusions:
- an infusion of plantain, approximately 20% by weight,
- an infusion of mallow, approximately 20% by weight,
- an infusion of camomile, approximately 20% by weight,
- an infusion of sage, approximately 10% by weight,
- an infusion of dog rose with java jute flowers, approximately 10% by weight,
- an infusion of orange flowers, approximately 10% by weight,
- an infusion of thyme, approximately 5% by weight,
with added lemon juice, approximately 5% by weight.

When, as already mentioned, a sweetening agent is added, the inventors suggest that honey should be used in order to obtain a mixture of perfectly natural substances.

It should also be pointed out that the inventors suggest that water (which may be distilled) should be used to prepare the infusions of the various herbs listed above, in which the following are placed in the infusion, respectively:
- approximately 1.5% by weight of plantain,
- approximately 0.9% by weight of mallow,
- approximately 0.9% by weight of camomile,
- approximately 1.5% by weight of sage,
- approximately 5.4% by weight of dog rose with java jute flowers,
- approximately 2.4% by weight of orange flowers
- approximately 1% of thyme.

Obviously the composition of the mixtures described above can be varied according to the desired therapeutic outcomes, also adding other types of infusion or altering the relative percentages of the various infused materials.

Finally it should be pointed out that the inventors are of the opinion that it is sufficient that the said cavity 3c in envelope 3 should have a volume of approximately 30 cm³ in order to obtain a bar 4 having the appropriate consistency and dimensions.

## Claims

1. Single-dose pack (1) of substances (2) capable of performing curative functions through absorption or contact with injured parts, **characterized in that** it comprises a sealed flexible envelope (3) of plastics material defining within it a cavity (3c) of elongated shape housing a single dose of the said substances (2) in the liquid state which when converted into the solid state by freezing adopt the shape of an elongated bar (4) of a length (L) such that it can be manipulated to reach the said injured parts with one end (4t).

2. Single-dose pack according to claim 1, in which the said substances (2) in the liquid state comprise a mixture of infused materials obtained from the following herbs:
- plantain,
- mallow,
- camomile,
- sage,
- thyme,
to which lemon juice is added.

3. Single-dose pack according to claim 2, in which the said substances (2) in the liquid state comprise a mixture of the following infused materials:
- an infusion of plantain, approximately 25% by weight,
- an infusion of mallow, approximately 25% by weight,
- an infusion of camomile, approximately 25% by weight,
- an infusion of sage, approximately 12.5% by weight,
- an infusion of thyme, approximately 6.25% by weight,
- with added lemon juice, approximately 6.25% by weight.

4. Single-dose pack according to claim 2, in which the said substances (2) in the liquid state comprise a mixture of the following infused materials:
- an infusion of plantain, approximately 20% by weight,
- an infusion of mallow, approximately 20% by weight,
- an infusion of camomile, approximately 20% by weight,
- an infusion of sage, approximately 10% by weight,
- an infusion of dog rose with java jute flowers, approximately 10% by weight,
- an infusion of orange flowers, approximately 10% by weight,
- an infusion of thyme, approximately 5% by weight,
- with added lemon juice, approximately 5% by weight.

5. Single-dose pack according to one of claims 2, 3, 4, to which a sweetening agent is added to the said substances (2) in the liquid state.

6. Single-dose pack according to claim 5, in which the said sweetening agent is honey.

7. Single-dose pack according to one of the foregoing claims, in which the infusions comprising the said mixture contain water in which the following are infused respectively:
- approximately 1.5% by weight of plantain,
- approximately 0.9% by weight of mallow,
- approximately 0.9% by weight of camomile,
- approximately 1.5% by weight of sage,
- approximately 5.4% by weight of dog rose with java jute flowers,
- approximately 2.4% by weight of orange flowers
- approximately 1% of thyme.

8. Container according to one of the foregoing claims in which the volume of the cavity (3c) housing a single dose is approximately 30 cm³.
